# EUROPEAN PATENT SPECIFICATION

(11) **EP 0 535 143 B1**
(45) Date of publication and mention of the grant of the patent: **23.08.1995**
(21) Application number: 91912546.8
(22) Date of filing: 26.06.1991
(51) Int. Cl.: B04B 5/04

(54) **METHOD AND MEANS FOR BLOOD SEPARATION**
VERFAHREN UND VORRICHTUNG ZUR BLUTTRENNUNG
PROCEDE ET DISPOSITIF DE SEPARATION DU SANG

(30) Priority: 26.06.1990 SE 9002255
(43) Date of publication of application: 07.04.1993
(73) Proprietor: OMEGA MEDICINTEKNIK AB, 115 29 Stockholm (SE)
(72) Inventor: UNGER, Peter, S-115 29 Stockholm (SE); WESTBERG, Eric, S-181 31 Lidingö (SE)
(74) Representative: Wiedemann, Bernd
(86) International application number: SE9100463
(87) International publication number: WO9200145

(56) References cited:
- WO-A-84/02091
- WO-A-87/06857
- US-A- 3 211 368
- US-A- 3 559 880
- US-A- 4 303 193
- US-A- 4 322 298
- US-A- 4 447 220

## Description

Conventionally, blood is separated into its various components by collecting blood batches in sterile plastic containers that contain an anticoagulant, to a total volume of about 500 ml, whereafter the containers, together with component containers connected thereto, are lowered into outwardly swingable cups in centrifuges and centrifuged until the blood present in the containers has been stratified in layers of cell concentrate, plasma and an intermediate layer, buffycoat, with a specific weight between the specifically heaviest component, the cell concentrate, and the specifically lightest component, the plasma, to the desired degree of purity. One serious problem with this conventional method resides in transferring the stratified components to the component containers connected to said blood container without slurrying or mixing the components.

US-A-4,303,193 discloses an apparatus for centrifugally separating blood into a first plasma rich component and a second plasma-poor component. Blood is introduced into a flexible blood processing bag designed to be supported within a contoured processing chamber in a centrifuge rotor so that the second blood component travels along a short internal bag dimension to achieve separation. The blood bag is arranged vertically during also during centrifugation. A displacement chamber having a fluid operated diaphragm is also positioned within the blood processing chamber in abutment with the side of the blood bag. The separated first blood component can be expressed from the flexible blood bag by movement of the diaphragm and collected in a receiver container as the rotor spins. The centrifuge rotor can then be stopped to allow return of the second blood component to the blood donor.

US-A-4,447,220 has regard to a cup of the centrifuge with two chambers. In the first chamber is placed a first blood bag filled with blood, which is inserted into one chamber together with an elasltically deformable inset cup. A pressure pad bears at one end of the outside of the inset cup and at the other end of the inside of the chamber of the centrifuge. During the centrifuging, the pressure pad is admitted with pressure which results in a great volume increase. The inset cup is thus compressed elastically so that the blood bag is compressed and the blood components centrifuged in the blood bag are transferred corresponding to the volume increase of the pressure pad into an empty satellite-blood bag arranged in the adjacent chamber. Over the upper connecting part of the blood bag projecting from the chamber is pushed a holder which prevents bending of the connecting tube during the centrifuging process.

When practicing conventional methods, the blood containers connected to the component containers are lifted carefully from the cups and placed in presses with the connected component containers positioned free from the press. According to the original method, which is the method most practiced today, the plasma is pressed first into the plasma container, through a hose connected to the top of the blood container, whereafter the buffycoat, when it enters the hose, is passed to a buffycoat container. The cell concentrate remains in the blood container. The drawbacks with this method are that re-slurrying of the blood components cannot be avoided when lifting the blood container from the centrifuge cup and placing the container in the press, and that considerable manual work is required in constantly monitoring the transfer process.

According to one alternative method, there is used a blood container which is provided with a bottom connection and which is placed in a press that is provided with a photocell at approximately the level of the buffycoat in the blood container when the blood container is placed in the press, in order to detect the interface between plasma and buffycoat. The process of pressing plasma and cell concentrate from the bag into respective component containers is controlled electronically in such a way that upon completion of the transfer process solely buffycoat will remain in the blood container, mixed with some of the other components. Although this method is labor-saving, since it is not necessary to monitor the transfer process, the transfer apparatus is several times as expensive as those normally used and the risk of re-slurrying of the blood components when removing the blood container from the centrifuge cup remains.

According to both methods the blood containers are usually not inserted directly into the centrifuge cups but in an insert therein. Since the blood container must be taken out of the insert this fact does not affect the given description.

One object of the present invention is to provide a method which will prevent re-slurrying of the stratified blood components prior to transferring said components to respective component containers.

Another object of the invention is to provide a cassette which can be used to accommodate the blood container during the process of centrifugation and thereafter to transfer at least one blood component to a component container in a sterile fashion and therewith to avoid re-slurrying.

Still another object of the invention is to provide a method for separating thrombocytes from the buffycoat interlayer obtained subsequent to centrifugation, and to transfer the thrombocytes to a component container intended herefor, in a sterile fashion.

These and other objects of the invention will be apparent from the following description.

The aforementioned drawbacks are alleviated in accordance with the invention in the following manner.

Blood contained in a collapsible container is separated into blood components by centrifugation, wherein, subsequent to the process of centrifugation, at least one of three stratified components is transferred in sterile fashion to a component container which is connected by means of an open or openable hose to the upper part of the container, and wherein the container is placed in a cassette that can be removed from the centrifuge, together with a fluid-actuable component displacement body which extends along and parallel with one side of said container.

According to one preferred embodiment, fluid is delivered to the displacement body and the displacement body is constructed such that when fluid is delivered thereto first the lower end of said body will expand, followed by gradual expansion of the body in an upward direction, thereby compressing the container first at its lower part and then successively in an upward direction, wherein the connection between said container and said component container is broken subsequent to having transferred a predetermined quantity of component, and optionally ceases with the delivery of said fluid.

According to another preferred embodiment in which the container is connected to a second component container by means of a second openable hose, the second hose includes a first extension which extends through a given distance in the container, taken from an upper container defining surface, down towards the bottom of the container, wherein, subsequent to breaking the connection between said container and said component container, the connection to the second component container is opened while continuing to deliver fluid to the displacement body, wherein the lower component layer is transferred to the second component container, and wherein the delivery of fluid ceases simultaneously or in sequence herewith when a second predetermined quantity of the lower component layer has been transferred to the second component container and the connection between the container and the component container is broken.

According to still another embodiment of the invention, the fluid is removed from the displacement body and a PSM-solution, i.e. a thrombocyte-suspending medium, is taken from a third component container and delivered to the intermediate fraction remaining in the container. This third component container is connected to the blood container via a third openable hose which opens into the first hose upstream of the connection-breaking location or into the upper end of the container, wherein the container is again centrifuged, this time at a low speed, and fluid is then delivered to the displacement body, so as to transfer an upper layer obtained by said blood centrifugation to the third component container, whereafter the supply of fluid is interrupted and the connection between said container and the third component container is broken.

According to an alternative method, blood is centrifuged in a collapsible container so as to separate the blood into component layers, and, subsequent to centrifugation, at least one of three stratified or layered components is transferred in sterile fashion to a component container which is connected to the upper part of the blood container by means of an open or openable hose, wherein the container is placed in a cassette which can be removed from the centrifuge, together with a fluid-actuable displacement body disposed along and parallel with one side of the blood container, wherein fluid is delivered to the displacement body, which has a construction such that when fluid is delivered thereto, said body will expand first at its lower end and then gradually upwards, such as to compress the container first at its lower end and then successively in an upwards direction; wherein the connection between the container and the component container is broken subsequent to having transferred a predetermined quantity of component to the component container and to the displacement body is optionally ceased with the delivery of fluid. In this case, the hose includes a second extension which extends in the container, towards the bottom thereof, through a second, predetermined distance which is greater than said first predetermined distance, for the transfer of the lower of said three component layers to the cell component container in response to fluid delivery.

When the blood container is connected to a plasma-component container by means of a second openable hose which opens into the upper defining surface of the blood container, the connection between said blood container and the cell-component container is broken and the connection to the plasma-component container is opened and delivery of fluid to the displacement body is continued. As a result, the upper component, i.e. the plasma, is transferred to the plasma-component container and the delivery of fluid is interrupted when a second predetermined quantity of plasma has been transferred to the plasma container, whereafter the connection between the blood container and the plasma-component container is broken.

Fluid is removed from the displacement body and a PSM-solution is taken from a third component container and added to the intermediate fraction that remains in the blood container, this third component container being connected to the blood container by means of a third openable hose which opens into the second hose upstream of the breaking location or into the upper end of the container, whereafter the blood container is again centrifuged, at a low speed, and fluid is again delivered to the displacement body so as to transfer the upper layer obtained by centrifugation to the third component container, whereafter the delivery of fluid is interrupted and the connection between the blood container and the third component container is broken.

The invention also relates to a device which can be placed in a centrifuge vessel and which functions to transfer, in a sterile fashion, at least one component in a stratified liquid obtained subsequent to centrifuging liquid (blood or blood fraction) present in a collapsible container, to a component container which is connected to the upper part of the blood container by means of an open or openable hose, said device including a container cassette and a fluid-actuable displacement body which is disposed parallel with and along one side of the container and which functions to reduce the container volume when fluid is delivered to said body, thereby effecting transfer of the container content to the component container in open communication with said blood container, wherein the displacement body is in direct abutment with the container, and wherein said device can be removed from the centrifuge.

The wall of the displacement body preferably has a resistance to expansion which varies in a manner such that displacement of the container contents will take place from the bottom of said container.

It is particularly preferred that the displacement body abuts the container over an area which is essentially equal to half the area of the container, and that the displacement body has a smallest wall thickness at its lower end and that said wall thickness increases with distance from said end.

According to another embodiment of the invention, only that side of the displacement body which faces the container has a varying wall thickness.

According to one embodiment, the displacement body is made of an elastic material of a kind which will return to its original form when no longer influenced by fluid pressure. The displacement body and the blood container may also have a common partition wall.

The blood container is placed in a surrounding, rigid or semi-rigid enclosure, i.e. a cassette, in which it is lowered into an outwardly swingable centrifuge cup and centrifuged, and is not removed from the cassette until the plasma and cell concentrate have been transferred to respective connected component containers. The components are transferred to respective component containers with the aid of an elastic bladder which can be pressurized and abuts, either directly or indirectly, the blood container during the process of centrifugation and component transfer. By pressurizing the bladder subsequent to centrifugation, the blood container is subjected to an overpressure which forces the intended parts of the container content through hoses connected to said container. The plasma is pressed into the plasma container through a connection provided at the top of the blood container. The cell concentrate can be pressed into its component container in a number of different ways. Firstly, the plasma content can be transferred through a hose which is connected internally to the top of the blood container and which extends to a level beneath the buffycoat layer formed during the process of centrifugation, and secondly, and also preferably, through a hose which is connected internally to the top of the blood container and which has a short length such as to prevent the hose from reaching the buffycoat layer formed during the process of centrifugation.

When practicing the first of these methods, it makes no real difference which of the aforesaid blood components is transferred first to its respective container, or whether the blood components are transferred simultaneously. When practicing the preferred method, however, it is necessary to transfer the plasma first. When the plasma is transferred to its respective container, in accordance with the present invention, the buffycoat layer will rise so that the mouth of the cell-concentrate hose will be located beneath the buffycoat layer. When subsequently the cell concentrate is transferred to its respective container, it will not contain buffycoat. The preferred method has the following advantages over the first mentioned method. The cell concentrate has a greater flow resistance and consequently it is important to use a short transfer hose, and the preferred method uses the shortest hose. In both methods the cell concentrate hose is filled with blood upon commencing the centrifugation process, if not earlier. When practicing the first method, a buffycoat layer is formed in the hoses during the process of centrifugation, this buffycoat layer subsequently being transferred to the cell-concentrate container and contaminating the cell concentrate. When practicing the preferred method, the blood drains from the hose during the process of centrifugation and is replaced with plasma which is free from buffycoat.

In addition to obtaining pure blood components, it is also highly important to be able to separate the largest possible quantities of said blood components in individual containers inexpensively. This can be effected in accordance with the present invention in the following manner, taking into account the particular simplicity of the equipment used. By placing the component containers in volume-limiting cassettes prior to the transfer of said components, the quantity to be transferred can be determined without the use of expensive electronic devices. However, the optimal quantities of the components that can be obtained from a blood unit is determined by the hematocrit of the blood (the cell concentration), which varies from one individual to another. The widest variation, however, depends on the sex of the blood donor, and a good result can be obtained by using separate cassettes for men and women (volumetrically adjustable).

Another advantage afforded by the inventive system is that blood can be drained or tapped at a constant volume without the use of expensive electronic, weight-constant blood-draining cradles. The volume-constant cassette in which the blood is centrifuged can also be used for blood-collecting purposes in combination with a simple rocking device.

The invention will now be described in more detail with reference to exemplifying embodiments thereof illustrated in the accompanying drawings, in which
Figure 1 is a front view of a container unit comprising a blood container provided with a blood-tapping connection and plasma and cell-concentrate containers connected to the blood container by means of hoses;
Figure 2 illustrates a collapsible cassette intended for a blood container and adapted to fit into an intermediate container (into an outwardly swingable centrifuge cup provided with a partition wall), and shows one cassette section provided internally with an elastic bladder that is fitted with a fluid connector;
Figure 3 illustrates the unit shown in Figure 1 with a full blood container in side view and with the container placed in a closed cassette according to Figure 2 and inserted in one of the compartments defined by an inner container equipped with a partition wall and adapted to fit into an outwardly swingable centrifuge cup, wherein the component containers are located in the other compartment;
Figure 4 illustrates the unit shown in Figure 3 subsequent to centrifugation and subsequent to having removed the inner container from the centrifuge cup and connecting the container to a pressure device, with the component containers placed outside the intermediate container;
Figure 5 illustrates a unit according to Figure 4 during the final stage of a component transfer operation, the blood container being provided with a long extension in the container;
Figure 6 is a front view of a container unit comprising a blood container fitted with a blood-draining connection and hose-connected cell-concentrate, plasma and thrombocyte containers; and
Figure 7 illustrates an intermediate container according to Figures 2-4, in which each compartment is divided into two subcompartments in which two blood bags are centrifuged simultaneously for thrombocyte separation.

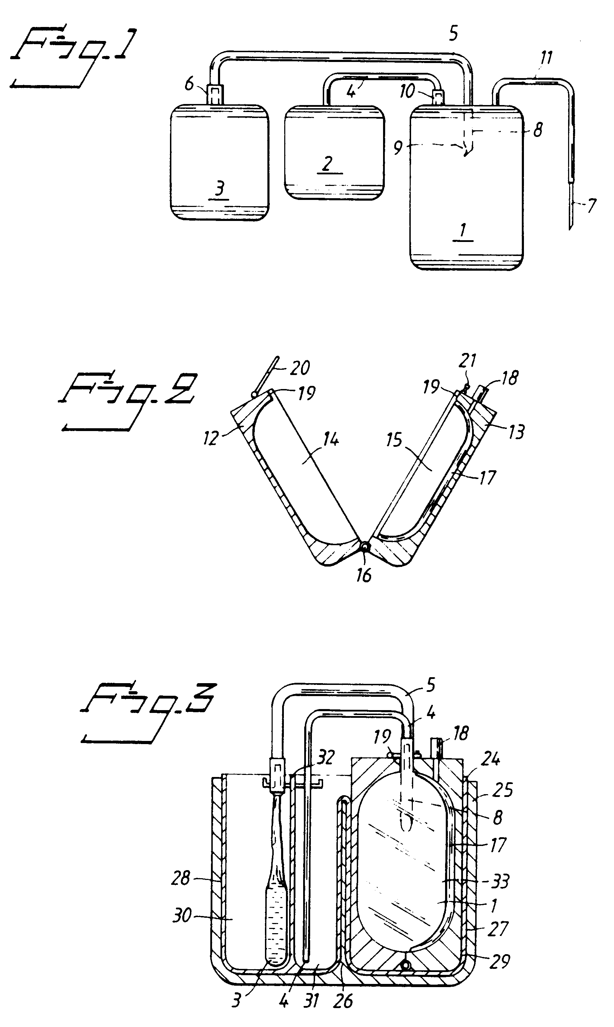

Figure 1 illustrates a container unit comprising a blood container 1 which contains an anticoagulant and which is connected to a blood-capping needle 7 by means of a hose 11. The blood container 1 is also connected by means of a top-connected hose 4 to a plasma-component container 2, the hose 4 being closed at a location adjacent the container 1 by means of an openable closure device 10, for example a shear pin. The upper part of the blood container 1 is connected to a cell-concentrate container 3 by means of a hose 5, said container containing a nutrient solution. The hose 5 includes an extension part 8 which extends into the blood container 1 and which has a side opening 9. The extension part 8 is preferably fairly rigid, although it may also consist of a thin, pliable material. The opening 9 may also be disposed in the longitudinal direction of the extension 8. The hose 5 is fitted with an openable closure device 6 adjacent the container 3, for example a shear pin similar to the closure device 10.

In Figure 2, the reference numerals 12 and 13 identify two cassette halves which are hinged together, e.g., by a hinge 16. The cassette half 13 is lined internally with an expandable displacement body 17, for instance a rubber bladder, which is provided with a fluid connector 18 in the upper part of the cassette holder 13. When assembled, the cassette halves define an inner space 14 + 15 which corresponds to a filled blood container 1 both in configuration and volume. The halves are locked together by means of a locking bar or strap 20 which engages a pin or stud 21. Provided in the upper defining surface of the cassette halves 12, 13 is an aperture 19 which accommodates the hoses connecting the blood container to the side containers, and optionally also other hoses.

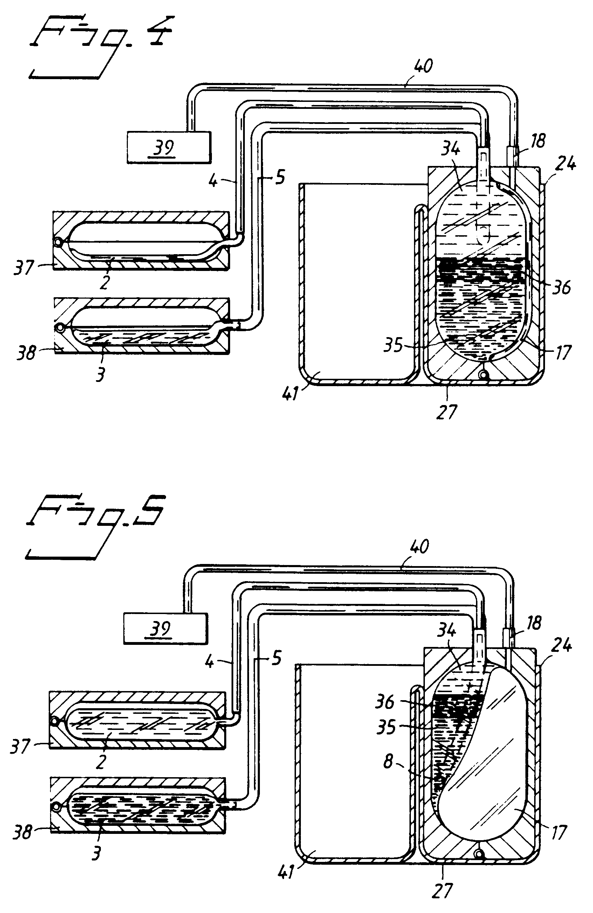

Figure 3 illustrates the unit shown in Figure 1 with the blood container 1 filled with a predetermined quantity of blood 33 and placed in the cassette 12, 13. The needle 7 has been welded-off and the end of the hose 11 (not shown) remote from the blood container has been closed. The cassette 12, 13 is placed in an intermediate container 24, which in turn is placed in a centrifuge cup 25. The centrifuge cup 25 is divided into two compartments 27, 28 by a partition wall 26. The intermediate container 24 has three compartments 29, 30, 31, wherein the shape and size of the cassette 12, 13 corresponds to the shape and size of the compartment 29 and the cassette is placed in this container compartment and located in the compartment 27 of the centrifuge cup. The compartments 30 and 31 of the intermediate containers are disposed in the compartment 28 of the centrifuge cup and are mutually separated by an intermediate wall 32. The plasma container is located in compartment 31 and the cell-concentrate container, containing said nutrient solution, is placed in compartment 30. The hoses connecting the blood container to respective side containers extend through the aperture 19 provided in the cassette 12, 13. The unit illustrated in Figure 3 is now ready for centrifugation, although has not yet undergone centrifugation.

Figure 4 illustrates the intermediate container 24 with its contents subsequent to having been removed from the centrifuge body 25, upon completion of the centrifugation process. The blood 33 has been divided by centrifugation into plasma 34, cell concentrate 35 and buffycoat 36. The side containers, or component containers 2, 3 that are intended for plasma and blood concentrate have been placed in separate respective compartments 37 and 38. A fluid source, for instance an air source, is connected to the connector 18 of the displacement body 17 by means of a hose 40. In this embodiment, the intermediate container 24 has only two compartments, i.e. compartment 29 for the cassette 12, 13 and compartment 41 for the component containers 2, 3. The source of fluid 39 may be an air compressor or a container for pressurized gas.

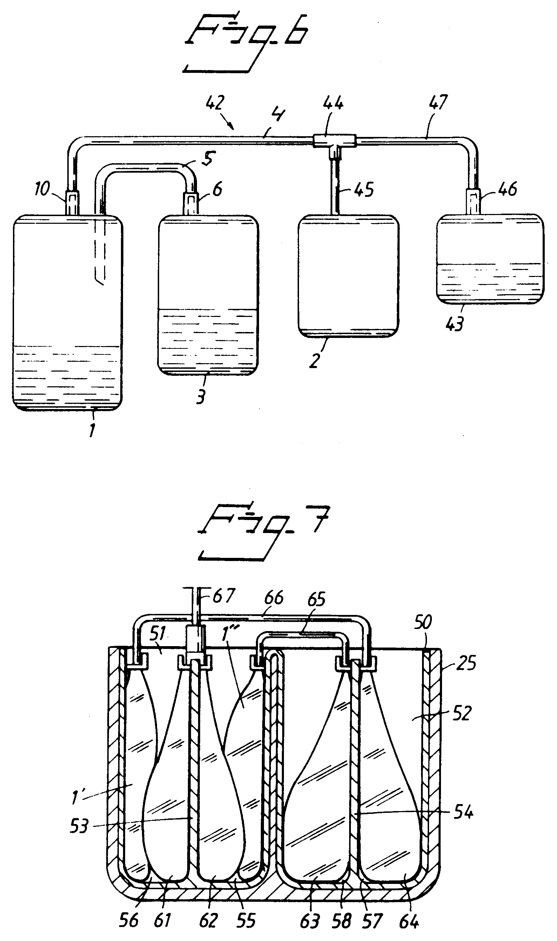

Figure 5 illustrates the same arrangement or unit as that shown in Figure 4, but with the exception that fluid has been delivered to the displacement body 17, such that the component holder 2 is filled with plasma and the component holder 3 is almost filled with cell concentrate. When fluid is delivered to the displacement body 17, the body first expands at its lower part and then continues to expand successively upwards. The intermediate layer 36, buffycoat and residues of plasma and cell concentrate, remain in the blood bag. The displacement body 17 is made of an elastic material, preferably a material which will return to its original shape and form when the fluid has been removed from the body. The body also has a thinnest wall thickness at its lower part, with the wall thickness of said body gradually increasing in value. Alternatively, the body 17 may be provided with parallel, transversely extending thinner parts which are disposed more densely in the lower part of the body and more sparsely in the upper part thereof. The displacement body 17 preferably encloses half the filled blood container 1 in the cassette 12, 13, optionally with the exception of the uppermost part of said container 1.

Figure 6 illustrates a container unit 42 which includes the container unit shown in Figure 1. The container unit 42 includes a thrombocyte container 43 which includes a thrombocyte-suspending medium. The container 43 is connected, by means of a hose 47, to a branch tube 44 mounted in the hose extending between the blood container and the plasma component container 2. In the case of this embodiment, the plasma container 2 is connected by means of a hose 45 to the branch tube 44, and the hose 5 is connected to the third outlet of the branch 2. An openable closure device is mounted in the hose 47 at a location adjacent the upper defining surface of the container 43. This closure device may be of the same kind as the closure devices 6 and 10 of the Figure 1 embodiment. The hose 11 by means of which blood is delivered to the container 1 is not shown. All hoses 4, 5, 11, 45 and 47 are made of a pliable material.

Figure 7 illustrates an intermediate container 50 inserted in the centrifuge cup 25. The intermediate container 50 has two compartments 51, 52, each of which is divided into two subcompartments 55, 56 and 57, 58 respectively by means of two partition walls 53, 54. Arranged in each subcompartment 55, 56 of the compartment 51 is a respective displacement body 61 and 62 and a respective buffycoat-containing blood bag 1' and 1''. A thrombocyte-component container 63, 64 is arranged in respective subcompartments 57, 58 of the compartment 52. The blood bag 1' is connected to the thrombocyte container 64 by means of a hose 66, while the blood bag 1'' is connected to the thrombocyte container 63 by means of hose 65. The displacement bodies 61, 62 are connected to a fluid source by means of a hose 67. The Figure illustrates the arrangement subsequent to completing a second centrifuging process at a lower speed than the first centrifuging process, and with the component containers for plasma and cell concentrate removed.

A unit of blood is drained or tapped into the blood container 1 in a conventional manner, said container containing a predetermined quantity of anticoagulant, whereafter the blood-tapping needle 7 is welded-off. The component container 3 for cell concentrate contains a predetermined quantity of nutrient solution. The blood container 1 is then placed in the cassette 12, 13, provided with the displacement body 17, and the cassette is closed and lowered into one compartment 29 of the intermediate container 24. The component containers 2 and 3 are placed, together with their connecting hoses, into two respective second compartments 31 and 30 in the intermediate container. The intermediate container 24, together with its contents, is then placed in the outwardly swingable centrifuge cup 25 of a centrifuge and then centrifuged. In order to achieve rapid and more effective separation of the blood components, it is beneficial to suspend the centrifuge cup eccentrically in the centrifuge, so that during a working revolution of the centrifuge, the centrifuge cup will form an angle of from 30 to 45 degrees with the vertical. Alternatively, the cassette 12, 13 can be constructed so that the blood bag will define the aforesaid angle during centrifugation. The blood 33 present in the blood container 1 is therewith divided into the components plasma 34, cell concentrate 35 and an intermediate buffycoat 36. The extension 8 shown in Figures 1, 3 and 4 and extending down in the container 1 is so short that its laterally directed orifice 9 will now be located in the plasma space.

When taking blood from a blood donor, the hose extension 8 will become partially filled with blood, but when the buffycoat layer is formed during the process of centrifugation, this blood will be caused to run from the extension, by the forces of centrifugation, and will be replaced with plasma. In order to utilize the centrifuge effectively, the intermediate container, with the cassette 12, 13 and component containers 2, 3, is lifted from the centrifuge body 25 and placed on a flat surface. A pressure source, for instance a compressed air source 39, is connected to the fluid connection 18 of the displacement body 17. The closure device 10 provided for opening the connection to the plasma container 2 is opened and fluid is then delivered to the displacement body 17. The displacement body 17 is so constructed, through its varying wall thickness, that said body will first compress the lower part of the blood container 1, the displacement body 17 being expanded from the bottom and upwards, as indicated in Figure 5. The container 1 is emptied so that the upper layer 34 (Figure 4), i.e. the plasma, flows into the plasma container 2. Subsequent to transferring a predetermined quantity of plasma, i.e. when the plasma container is full, the closure device 10 of the cell concentrate container 3 is opened, this container containing nutrient solution for the blood cells. As the plasma is displaced upwards in the container 1, the buffycoat layer 36 will pass the orifice 9 in the extension line 8. As the closure device is opened, any connection to the plasma container is closed, e.g. with the aid of a hose clamp. When switching from plasma to cell concentrate, the delivery of fluid may be interrupted temporarily. When delivery of said fluid is continued, cell concentrate, which is now located around the orifice 9 of the extension 8, will flow over into the container 3. Subsequent to having transferred said predetermined quantity of cell concentrate and the concentrate container is full, the delivery of fluid is interrupted and the connection between the containers 1 and 3 is broken. The component containers 2 and 3 are then removed from the intermediate container 24 and the hoses 4 and 5 are welded-off and sealed permanently. The cassette containing the blood container 1, which now contains solely buffycoat and residues of plasma and cell concentrate, is removed from the cassette for subsequent separation of thrombocytes. The afore described procedure can also be carried out in the centrifuge cup, although with the disadvantage that in that case the centrifuge cannot be used during the transfer.

According to one alternative method, the intermediate container 24 is removed from the centrifuge cup 25 upon completion of the centrifugation process and placed on a supporting surface. Instead of being left in the intermediate container 24, the component containers are removed and placed into separate compartments, as shown in Figures 4 and 5.

The embodiment illustrated in Figure 5 differs from the embodiments shown in Figures 2, 3 and 4, in that the blood bag has an extension 70 which extends far into the blood container 1, at least to a location beneath the buffycoat layer 36 formed upon centrifugation. The transfer of the blood-components plasma and cell concentrate can therewith be effected in the same order or in the reverse order, or even at one and the same time.

The container unit 42 illustrated in Figure 6 is used when thrombocytes are separated from buffycoat in conjunction with transferring plasma and cell concentrate to their respective containers 2, 3. Upon completion of the plasma and cell-concentrate transfer, the hose 45, which connects the plasma container with the branch pipe 44, is welded-off instead of the hose 5. The thrombocyte-suspending medium, the PSM-solution, in the container 43 is delivered to the blood container 1, and the blood container 1 is placed in the intermediate container 50, in accordance with Figure 7. In the case of the illustrated embodiment, two blood containers 1 can be placed in respective subcompartments 55, 56, whereas the associated component containers 43 are placed in respective subcompartments 57 and 58. A further centrifugation process is carried out subsequent to having placed the intermediate container in the centrifuge body 24, although this time at a lower speed than that at which the first centrifugation process was carried out. By lower speed is meant in the present description and claims that the blood container is subjected to less g-forces to which the container is subjected. Subsequent to centrifugation, a displacement sequence similar to that described with reference to Figures 3 and 4 in the case of plasma displacement takes place.

## Claims

1. A method of separating blood into components by centrifuging blood present in a collapsible container (1), and in sterile fashion transferring at least two of three components stratified by said centrifugation process to separate side containers (2, 3) connected to the upper part of the blood container (1) by means of a first open or openable hose (4) and a second openable hose (5), respectively, wherein the second hose (5) includes an extension part (8) which extends towards the container (1) bottom through a predetermined distance, taken from the upper defining surface of said container (1), wherein the orifice (9) of said extension part (8) is located either above or below the center layer (36) of said three layers (34, 35, 36) subsequent to centrifugation and breaking the connection between the blood container (1) and the side containers (2, 3) subsequent to the transfer of the blood component to the respective side container (2, 3), **characterized** by the steps of
placing, before centrifugation, the blood container (1) and a fluid actuable displacement body (17) in a cassette (12, 13) enclosing said container (1) and displacement body (17) and having an opening for a fluid connection to the displacement body (17) and the hoses (4, 5) connecting the blood container (1) with the side containers (2, 3), said displacement body (17) being arranged in direct contact with the container (1), along and parallel with one side thereof;
actuating, after centrifugation, the displacement body (17) with a fluid in order to transfer the lighter component (34) to the first side container (2), in case of an openable hose, after opening the connection between the container (1) and the side container (2);
opening the connection between the blood container (1) and the second side container (3) subsequent to the completion of the transfer of the lighter component (34);
continuing the delivery of fluid to the displacement body (17) such as to transfer the heavier component (35) to the second side container (3);
interrupting the delivery of fluid when a second predetermined quantity of the heavier component has been transferred to the second side container (3).

2. A method according to Claim 1, **characterized** in that said displacement (17) body is given such a construction that, when fluid is delivered thereto, said body (17) will expand first at its lower end and then gradually upwards, whereby the container (1) is compressed first at its lower part and such that said compression will propagate in an upward direction.

3. A method according to Claim 2, **characterized** by removing the fluid from the displacement body (17); adding a PSM-solution, i.e. a thrombocyte-suspending medium, to the remaining intermediate fraction in said container (1), said PSM-solution being taken from a third side container (43) which is connected to said container (1) by means of a third openable hose (47) which opens into the first hose (4) upstream of the location at which said connection is broken, or opens into the upper end of said container (1); centrifuging the container (1) again, at a low speed; delivering fluid to the displacement body (17) such as to transfer the upper layer obtained by centrifugation to the third side container (43); interrupting the delivery of fluid; and breaking the connection between the container and the third side container.

4. A method of separating blood into components by centrifuging blood present in a collapsible container, and in sterile fashion transferring at least two of three components (34, 35, 36) stratified by said centrifugation process to separate side containers (2, 3) connected to the upper part of the blood container (1) by means of a first open or openable hose (4) and a second openable hose (5), respectively, wherein the second hose (5) includes an extension part (8) which extends towards the container (1) bottom through a predetermined distance, taken from the upper defining surface of said container (1), wherein the orifice (9) of said extension part (8) is located below the center layer (36) of said three layers (34, 35, 36) subsequent to centrifugation and breaking the connection between the blood container (1) and the side containers (2, 3) subsequent to the transfer of the blood component to the respective side container (2, 3), **characterized** by the steps of
placing, before centrifugation, the blood container (1) and a fluid actuable displacement body (17) in a cassette (12, 13) enclosing said container (1) and displacement body (17) and having an opening for a fluid connection to the displacement body (17) and the hoses (4, 5) connecting the blood container (1) with the side containers (2, 3), said displacement body (17) being arranged in direct contact with the container (1), along and parallel with one side thereof;
opening, after centrifugation, the second connection (6) and in case of an open first hose (4) closing the first connection (10) and actuating the displacement body (17) with a fluid in order to transfer the heavier component (35) to the second side container (3);
opening the connection between the blood container (1) and the first side container (2) subsequent to the completion of the transfer of the heavier component;
continuing the delivery of fluid to the displacement body (17) such as to transfer the lighter component (34) to the first side container (2);
interrupting the delivery of fluid when a second predetermined quantity of the lighter component (34) has been transferred to the first side container (2).

5. A method according to Claim 1, **characterized** in that said displacement body (17) is given such a construction that, when fluid is delivered thereto, said body (17) will expand first at its lower end and then gradually upwards, whereby the container (1) is compressed first at its lower part and such that said compression will propagate in an upward direction.

6. A method according to Claim 2, **characterized** by removing the fluid from the displacement body (17); adding a PSM-solution, i.e. a thrombocyte-suspending medium, to the remaining intermediate fraction (36) in said container (1), said PSM-solution being taken from a third side container (43) which is connected to said container (1) by means of a third openable hose (47) which opens into the first hose (4) upstream of the location at which said connection (46) is broken, or opens into the upper end of said container (1); centrifuging the container again, at a low speed; delivering fluid to the displacement body (17) such as to transfer the upper layer obtained by centrifugation to the third side container (43); interrupting the delivery of fluid; and breaking the connection between the container (1) and the third side container (43).

7. A method of separating blood into components by centrifuging blood present in a collapsible container (1), and in sterile fashion transferring at least two of three components (34, 35, 36) stratified by said centrifugation process to separate side containers (2, 3) connected to the upper part of the blood container (1) by means of a first open or openable hose (4) and a second open-able hose (5), respectively, wherein the second hose (5) includes an extension part (8) which extends towards the container (1) bottom through a predetermined distance, taken from the upper defining surface of said container (1), wherein the orifice (9) of said extension part (8) is located below the center layer (36) of said three layers (34, 35, 36) subsequent to centrifugation and breaking the connection between the blood container (1) and the side containers (2, 3) subsequent to the transfer of the blood component to the respective side container (1), **characterized** by the steps of
placing, before centrifugation, the blood container (1) and a fluid actuable displacement body (17) in a cassette (12, 13) enclosing said container (1) and displacement body (17) and having an opening for a fluid connection to the displacement body (17) and the hoses (4, 5) connecting the blood container (1) with the side containers (2, 3), said displacement body (17) being arranged in direct contact with the container (1), along and parallel with one side thereof;
opening, after centrifugation, the second connection (6) and in case of an openable first hose (4) also the first connection (10) and actuating the displacement body (17) with a fluid in order to simultaneously transfer the heavier and lighter component to the respective side container (2, 3).

8. A device which can be placed in a centrifuge vessel for centrifugation and thereafter sterile transfer of at least two components of a stratified liquid obtained subsequent to centrifuging a liquid (blood or blood fraction) present in a collapsible container (1) to side containers (2, 3) connected to the upper part of the collapsible container (1) by means of hoses (4, 5), **characterized** in that said device includes a rigid or semi-rigid container cassette (12, 13) enclosing said container (1) and a fluid-actuable displacement body (17) being arranged in direct abutment, parallel with and along one side of the container (1), said cassette (12, 13) having an opening for connections to the displacement body (17) and the container (1) with its side containers (2, 3), in that one of the connections of the collapsible container (1) includes an extension part (8) which extends towards the container (1) bottom through a predetermined distance, taken from the upper defining surface of said container (1), and in that the device can be removed from the centrifuge before the transfer of the components from the container (1).

9. A device according to Claim 8, **characterized** in that the area over which the displacement body (17) abuts the blood container is substantially equal to half the area of said container.

10. A device according to Claim 8 or 9, **characterized** in that the resistance of the wall of the displacement body (17) to expansion varies in a manner such that displacement of the blood container (1) contents begins from the bottom of said container.

11. A device according to one more of Claims 8-10, **characterized** in that the displacement body (17) has a thinnest wall thickness at its lower end and in that said wall thickness increases with distance from said end.

12. A device according to one or more of Claims 8-11, **characterized** in that only the side of the displacement body (17) that faces the blood container has a varying wall thickness.

13. A device according to one or more of Claims 8-12, **characterized** in that the displacement body (17) is made of an elastic material of a kind which will return to its original form when no longer influenced by fluid pressure.

14. A device according to one or more of Claims 8-13, **characterized** in that the displacement body (17) and the container (1) have a common defining wall.

## Patentansprüche

1. Verfahren zum Trennen von Blut in Komponenten durch Zentrifugieren des in einem zusammenlegbaren Behälter (1) vorhandenen Blutes und steriles Überführen von wenigstens zwei von drei durch den Zentrifugierungsprozess stratifizierten Komponenten, in getrennte Seitenbehälter (2,3), die jeweils mittels eines ersten offenen oder zu öffnenden Schlauches (4) und eines zweiten zu öffnenden Schlauches (5) an den oberen Teil des Blutbehälters (1) angeschlossen sind, wobei der zweite Schlauch (5) einen Verlängerungsteil (8) aufweist, der sich über eine vorbestimmte Entfernung, gemessen von der oberen begrenzenden Oberfläche des Behälters (1), in Richtung zum Boden des Behälters (1) erstreckt, wobei sich die Austrittsöffnung (9) des Verlängerungsteils (8) nach der Zentrifugierung entweder über oder unter der mittleren Schicht (36) der drei Schichten (34,35,36) befindet sowie durch Trennen der Verbindung zwischen dem Blutbehälter (1) und den Seitenbehältern (2,3) nach der Überführung der Blutkomponenten in die jeweiligen Seitenbehälter (2,3), gekennzeichnet durch die Schritte:
vor der Zentrifugierung wird der Blutbehälter (1) und ein durch Fluid betreibbarer Verdrängungskörper (17) in eine den Behälter (1) und den Verdrängungskörper (17) umschließende Kassette (12,13) eingesetzt, und die eine Öffnung für einen Fluidanschluß an den Verdrängungskörper (17) und für die Schläuche (4,5) aufweist, die den Blutbehälter (1) mit den Seitenbehältern (2,3) verbinden, wobei der Verdrängungkörper (17) in direktem Kontakt mit dem Behälter (1), entlang und parallel zu einer Seite desselben, angeordnet ist;
nach der Zentrifugierung wird der Verdrängungskörper (17) durch ein Fluid betätigt, um die leichtere Komponente (34) in den ersten Seitenbehälter (2) zu überführen, nachdem im Falle eines zu öffnenden Schlauches die Verbindung zwischen dem Behälter (1) und dem Seitenbehälter (2) geöffnet wurde;
nach der Vollendung der Überführung der leichteren Komponente (34) wird die Verbindung zwischen dem Blutbehälter (1) und dem zweiten Seitenbehälter (3) geöffnet;
die Fluidzufuhr zu dem Verdrängungskörper (17) wird derart fortgesetzt, daß die schwerere Komponente (35) in den zweiten Seitenbehälter (3) überführt wird;
die Fluidzufuhr wird unterbrochen, wenn eine zweite vorbestimmte Menge der schwereren Komponente in den zweiten Seitenbehälter (3) überführt worden ist.

2. Verfahren nach Anspruch 1, dadurch gekennzeichnet, daß der Verdrängungskörper (17) derart ausgeführt ist, daß, wenn demselben Fluid zugeführt wird, sich der Körper (17) zuerst an seinem unteren Ende und dann nach und nach aufwärtsgerichtet ausdehnt, wodurch der Behälter (1) zuerst an seinem unteren Teil und derart zusammengedrückt wird, daß sich die Zusammendrückung in einer aufwärts gerichteten Richtung fortpflanzt.

3. Verfahren nach Anspruch 2, dadurch gekennzeichnet, daß das Fluid aus dem Verdrängungskörper (17) entfernt wird; daß eine PSM-Lösung, d. h. ein Thrombozyten suspendierendes Medium, zu dem in dem Behälter (1) verbleibenden Zwischenanteil hinzugegeben wird, wobei die PSM-Lösung aus einem dritten Seitenbehälter (43) entnommen wird, der an den Behälter (1) mittels eines dritten zu öffnenden Schlauches (47) angeschlossen ist, welcher stromaufwärts der Stelle, an der die Verbindung getrennt wird, in den ersten Schlauch (4), oder in das obere Ende des Behälters (1) mündet; erneutes Zentrifugieren, daß der Behälter (1) bei einer niedrigen Geschwindigkeit erneut zentrifugiert wird; daß Fluid zu dem Verdrängungskörper (17) derart zugeführt wird, daß die durch Zentrifugierung erhaltene obere Schicht in den dritten Seitenbehälter (43) überführt wird; daß die Fluidzufuhr unterbrochen wird; und daß die Verbindung zwischen dem Behälter und dem dritten Seitenbehälter getrennt wird.

4. Verfahren zum Trennen von Blut in Komponenten durch Zentrifugieren von in einem faltbaren Behälter vorhandenem Blut und steriles Überführen von mindestens zwei von drei durch den Zentrifugierungsprozess stratifizierten Komponenten (34,35,36) in getrennte Seitenbehälter (2,3), die an den oberen Teil des Blutbehälters (1) jeweils mittels eines ersten offenen oder zu öffnenden Schlauches (4) und eines zweiten zu öffnenden Schlauches (5) angeschlossen sind, wobei der zweite Schlauch (5) einen Verlängerungsteil (8) aufweist, der sich in Richtung zum Boden des Behälters (1) über eine vorbestimmte Strecke, gemessen von der oberen abgrenzenden Oberfläche des Behälters (1), erstreckt, wobei die Austrittsöffnung (9) des Verlängerungsteils (8) sich nach der Zentrifugierung unterhalb der mittleren Schicht (36) der drei Schichten (34,35,36) befindet, sowie durch Trennen der Verbindung zwischen dem Blutbehälter (1) und den Seitenbehältern (2,3) nach der Überführung der Blutkomponenten in die jeweiligen Seitenbehälter (2,3), gekennzeichnet durch die Schritte:
vor der Zentrifugierung wird der Blutbehälter (1) und ein durch Fluid betreibbarer Verdrängungskörper (17) in eine den Behälter (1) und den Verdrängungskörper (17) umschließende Kassette (12,13) eingesetzt, die eine Öffnung für einen Fluidanschluß an den Verdrängungskörper (17) und für die Schläuche (4,5) aufweist, die die Blutbehälter (1) mit den Seitenbehältern (2,3) verbinden, wobei der Verdrängungskörper (17) in direktem Kontakt mit dem Behälter (1), entlang und parallel zu einer Seite desselben, angeordnet ist;
nach der Zentrifugierung wird die zweite Verbindung (6) geöffnet, und im Falle eines offenen ersten Schlauches (4) Schließen die erste Verbindung (10) geschlossen und der Verdrängungskörpers (17) wird durch ein Fluid betätigt, um die schwerere Komponente (35) in den zweiten Seitenbehälter (3) zu überführen;
die Verbindung zwischen dem Blutbehälter (1) und dem ersten Seitenbehälter (2) wird nach der Vollendung der Übertragung der schwereren Komponente geöffnet;
die Fluidzufuhr zu dem Verdrängungskörper (17) wird derart fortgesetzt, daß die leichtere Komponente (34) in den ersten Seitenbehälter (2) überführt wird;
die Fluidzufuhr wird unterbrochen, wenn eine zweite vorbestimmte Menge der leichteren Komponente (34) in den ersten Seitenbehälter (2) überführt worden ist.

5. Verfahren nach Anspruch 1, dadurch gekennzeichnet, daß der Verdrängungskörper (17) derart ausgelegt wird, daß, wenn demselben Fluid zugeführt wird, der Körper (17) sich zuerst an seinem unteren Ende und dann nach und nach aufwärtsgerichtet ausdehnt, wobei der Behälter (1) zuerst an seinem unteren Teil und derart zusammengedrückt wird, daß sich das Zusammendrucken in eine aufwärts gerichtete Richtung fortpflanzt.

6. Verfahren nach Anspruch 2, dadurch gekennzeichnet, daß das Fluid aus dem Verdrängungskörper (17) entfernt wird; daß eine PSM-Lösung, d. h. ein Thrombozyten suspendierendes Medium zu der in dem Behälter (1) verbleibenden Zwischenfraktion (36) hinzugegeben wird, wobei die PSM-Lösung aus einem dritten Seitenbehälter (43) entnommen wird, welcher an den Behälter (1) mittels eines dritten zu öffnenden Schlauches (47) angeschlossen ist, der stromaufwärts der Stelle, an der die Verbindung (46) getrennt ist, in den ersten Schlauch (4) oder in das obere Ende des Behälters (1) mündet; daß der Behälter bei niedriger Geschwindigkeit erneut zentrifugiert wird; daß Fluid zu dem Verdrängungskörper (17) derart zugeführt wird, daß die durch Zentrifugierung erhaltene obere Schicht in den dritten Seitenbehälter (43) überführt wird; daß die Fluidzufuhr Unterbrochen wird; und daß die Verbindung zwischen dem Behälter (1) und dem dritten Seitenbehälter (43) getrennt wird.

7. Verfahren zum Trennen von Blut in Komponenten durch Zentrifugieren von in einem faltbaren Behälter (1) vorhandenem Blut und zum sterilen Übertragen von mindestens zwei von drei durch den Zentrifugierungsprozess stratifizierten Komponenten (34,35,36) in getrennte Seitenbehälter (2,3), die an den oberen Teil des Blutbehälters (1) jeweils mittels eines ersten offenen oder zu öffnenden Schlauches (4) und eines zweiten zu öffnenden Schlauches (5) angeschlossen sind, wobei der zweite Schlauch (5) einen Verlängerungsteil (8) aufweist, der sich über eine vorbestimmte Strecke, gemessen von der oberen begrenzenden Oberfläche des Behälters (1), in Richtung zum Boden des Behälters (1) erstreckt, wobei sich die Austrittsöffnung (9) des Verlängerungsteils (8) nach der Zentrifugierung unter der mittleren Schicht (36) der drei Schichten (34,35,36) befindet, sowie durch Trennen der Verbindung zwischen dem Blutbehälter (1) und den Seitenbehältern (2,3) nach dem Überführen der Blutkomponenten in den jeweiligen Seitenbehälter, gekennzeichnet durch die Schritte:
vor der Zentrifugierung werden der Blutbehälter (1) und ein durch Fluid betreibbarer Verdrängungskörper (17) in eine den Behälter (1) und den Verdrängungskörper (17) umgebende Kassette (12,13) eingesetzt, die eine Öffnung für einen Fluidanschluß an den Verdrängungskörper (17) und für die Schläuche (4,5), die den Blutbehälter (1) mit den Seitenbehältern (2,3) verbinden, aufweist, wobei der Verdrängungskörper (17) in direktem Kontakt mit dem Behälter (1), entlang und parallel zu einer Seite desselben, angeordnet ist;
nach der Zentrifugierung wird die zweite Verbindung (6) und im Falle eines ersten zu öffnenden Schlauches (4) auch die erste Verbindung (10) geöffnet und wird der Verdrängungskörper (17) durch ein Fluid betätigt, um die schwerere und die leichtere Komponente gleichzeitig in den jeweiligen Seitenbehälter (2,3) zu überführen.

8. Einrichtung, die in ein Zentrifugengefäß eingesetzt werden kann, zur Zentrifugierung und zur anschließenden sterilen Überführung von mindesten zwei Komponenten einer stratifizierten Flüssigkeit, die nach Zentrifugieren einer in einem faltbaren Behälter (1) enthaltenen Flüssigkeit (Blut oder Blutanteil) erhalten wurde, in Seitenbehälter (2,3), die durch Schläuche (4,5) an den oberen Teil des faltbaren Behälters (1) angeschlossen sind, dadurch gekennzeichnet, daß die Einrichtung eine starre oder halbstarre Behälterkassette (12,13) aufweist, die den Behälter (1) und einen durch Fluid betreibbaren Verdrängungskörper (17) umschließt, der direkt anliegend, parallel zu und entlang einer Seite des Behälters (1) angeordnet ist, wobei die Kassette (12,13) eine Öffnung für Verbindungen zu dem Verdrängungskörper (17) und des Behälters (1) mit seinen Seitenbehältern (2,3) aufweist, daß eine der Verbindungen des faltbaren Behälters (1) einen Verlängerungsteil (8) aufweist, der sich über eine vorbestimmte Entfernung, gemessen von der oberen begrenzenden Oberfläche des Behälters (1), in Richtung zum Boden des Behälters (1) erstreckt, und daß die Einrichtung vor der Überführung der Komponenten aus dem Behälter (1) aus der Zentrifuge entnommen werden kann.

9. Einrichtung nach Anspruch 8, dadurch gekennzeichnet, daß die Fläche, über die der Verdrängungskörper (17) an dem Blutbehälter anliegt, im wesentlichen gleich der Hälfte der Fläche des Behälters ist.

10. Einrichtung nach Anspruch 8 oder 9, dadurch gekennzeichnet, daß der Widerstand der Wand des Verdrängungskörpers (17) gegen Ausdehnung in einer Art und Weise derart variiert, daß die Verdrängung des Inhalts des Blutbehälters (1) von dem Boden des Behälters beginnt.

11. Einrichtung nach einem oder mehreren der Ansprüche 8-10, dadurch gekennzeichnet, daß der Verdrängungskörper (17) eine dünnste Wanddicke an seinem unteren Ende aufweist, und daß die Wanddicke mit Abstand von dem Ende zunimmt.

12. Einrichtung nach einem oder mehreren der Ansprüche 8-11, dadurch gekennzeichnet, daß nur die dem Blutbehälter zugekehrte Seite des Verdrängungskörpers (17) eine variierende Wanddicke aufweist.

13. Einrichtung nach einem oder mehreren der Ansprüche 8-12, dadurch gekennzeichnet, daß der Verdrängungskörper (17) aus einem elastischen Material einer Art hergestellt wird, die wieder ihre Ausgangsform annimmt, wenn dieselbe nicht mehr durch Fluiddruck beeinflußt wird.

14. Einrichtung nach einem oder mehreren der Ansprüche 8-13, dadurch gekennzeichnet, daß der Verdrängungskörper (17) und der Behälter (1) eine gemeinsame begrenzende Wand aufweisen.

## Revendications

1. Procédé pour diviser le sang en constituants en centrifugeant du sang présent dans un récipient compressible (1) et en transférant dans un mode stérile au moins deux de trois constituants stratifiés par ledit processus de centrifugation dans des récipients latéraux séparés (2, 3) respectivement raccordés à la partie supérieure du récipient de sang (1) au moyen d'un premier tuyau ouvert ou povant être ouvert (4) et d'un deuxième tuyau pouvant être ouvert (5), le deuxième tuyau (5) comprenant une partie prolongatrice (8) qui se prolonge vers le fond du récipient (1) sur une distance prédéterminée, mesurée à partir de la surface supérieure limite dudit récipient (1), et l'orifice (9) de ladite partie prolongatrice (8) étant placé, soit au-dessus, soit au-dessous de la couche centrale (36) des trois couches (34, 35, 36) précitées qui existent après la centrifugation, et en interrompant la communication entre le récipient de sang (1) et lesdits récipients latéraux (2, 3) après le transfert du constituant du sang au récipient latéral respectif (2, 3), caractérisé par les phases consistant à :
avant la centrifugation, placer le récipient de sang (1) et un corps de déplacement (17) pouvant être actionné par fluide dans une cassette (12, 13) qui renferme ledit récipient (1) et ledit corps de déplacement (17) et présente une ouverture permettant d'établir une liaison fluidique avec le corps de déplacement (17) et les tuyaux (4, 5) reliant le récipient de sang (1) aux récipients latéraux (2, 3), ledit corps de déplacement (17) étant disposé en contact direct avec le récipient, le long d'un côté de celui-ci et parallèlement à ce côté ;
après centrifugation, actionner le corps de déplacement (17) avec un fluide pour transférer le constituant léger (34) au premier récipient latéral (2), après l'ouverture de la liaison entre le récipient (1) et le récipient latéral (2) dans le cas d'un tuyau pouvant être ouvert ;
ouvrir la liaison entre le récipient de sang (1) et le deuxième récipient latéral (3) après avoir terminé le transfert du constituant léger (34) ;
continuer à envoyer du fluide au corps de déplacement (17) de manière à transférer le constituant lourd (35) au deuxième récipient latéral (3) ;
interrompre l'envoi de fluide lorsqu'une deuxième quantité prédéterminée du constituant lourd a été transférée au deuxième récipient latéral (3).

2. Procédé selon la revendication 1, caractérisé en ce qu'on donne audit corps de déplacement (17) une construction telle que, lorsque du fluide y est envoyé, ledit corps (17) se dilate tout d'abord à son extrémité inférieure, puis progressivement en remontant, de sorte que le récipient (1) est comprimé tout d'abord dans sa partie inférieure et de manière que ladite compression se propage dans une direction ascendante.

3. Procédé selon la revendication 2, caractérisé en ce qu'on évacue le fluide du corps de déplacement (17) ; on ajoute une solution PSM, par exemple un milieu de suspension des thrombocytes, à la fraction intermédiaire restante contenue dans ledit récipient (1), ladite solution PSM étant prélevée dans un troisième récipient latéral (3) qui est relié audit récipient (1) au moyen d'un troisième tuyau pouvant être ouvert (47) et qui débouche dans le premier tuyau (4) en amont du point où ladite liaison est interrompue, ou qui débouche dans l'extrémité supérieure dudit récipient (1) ; on centrifuge de nouveau le récipient (1), à basse vitesse ; on envoie du fluide au corps de déplacement (17) de manière à transférer la couche supérieure obtenue par centrifugation au troisième récipient latéral (43) ; on interrompt l'envoi du fluide et on interrompt la liaison entre le récipient et le troisième récipient latéral.

4. Procédé pour diviser le sang en ses constituants en centrifugeant du sang présent dans un récipient compressible et en transférant de façon stérile au moins deux des trois constituants (34, 35, 36) stratifiés par ledit processus de centrifugation dans des récipients latéraux séparés (2, 3) respectivement raccordés à la partie supérieure du récipient de sang (1) au moyen d'un premier tuyau ouvert ou pouvant être ouvert (4) et d'un deuxième tuyau pouvant être ouvert (5), dans lequel le deuxième tuyau (5) comprend une partie prolongatrice (8) qui se prolonge vers le fond du récipient sur une distance prédéterminée, mesurée à partir de la surface limite supérieure dudit récipient (1), dans lequel l'orifice (9) de ladite partie prolongatrice (8) est placé au-dessous de la couche centrale (36) des trois couches (34, 35, 36) qui existent après la centrifugation, et on interrompt la liaison entre le récipient de sang (1) et le récipient latéral (2, 3) après le transfert du constituant du sang un récipient latéral respectif (2, 3), caractérisé par les phases consistant à :
avant la centrifugation, placer le récipient de sang (1) et un corps de déplacement (17) pouvant être actionné par fluide dans une cassette (12, 13) qui renferme ledit récipient (1) et ledit corps de déplacement (17) et ayant une ouverture pour établir une liaison fluidique avec le corps de déplacement (17) et les tuyaux (4, 5) reliant le récipient de sang (1) aux récipients latéraux (2, 3), ledit corps de déplacement (17) étant disposé en contact direct avec le récipient (1), le long d'un côté de celui-ci et parallèlement à ce côté ;
après centrifugation, ouvrir la deuxième liaison (6) dans le cas d'un premier tuyau ouvert (4) en fermant la première liaison (10) et actionner le corps de déplacement (17) avec un fluide pour transférer le constituant lourd (35) au deuxième récipient latéral (3) ;
ouvrir la liaison entre le récipient de sang (1) et le premier récipient latéral (2) après l'exécution du transfert du constituant lourd (34) ;
continuer à envoyer du fluide au corps de déplacement (17) de manière à transférer le constituant léger (35) au deuxième récipient latéral (2) ;
interrompre l'envoi de fluide lorsqu'une deuxième quantité prédéterminée du constituant léger a été transférée au premier récipient latéral (2).

5. Procédé selon la revendication 1, caractérisé en ce que ledit corps de déplacement (17) présente une construction telle que, lorsque du fluide y est envoyé, ledit corps (17) se dilate tout d'abord à son extrémité inférieure, puis se dilate progressivement en remontant, de sorte que le récipient (1) est comprimé tout d'abord dans sa partie inférieure et de manière que ladite compression se propage dans une direction ascendante.

6. Procédé selon la revendication 2, caractérisé en ce qu'on évacue le fluide du corps de déplacement (17) ; on ajoute une solution PSM, par exemple un milieu de suspension des thrombocytes, à la fraction intermédiaire restante (36) contenue dans ledit récipient (1), ladite solution PSM étant prélevée dans un troisième récipient latéral (3) qui est relié audit récipient (1) au moyen d'un troisième tuyau pouvant être ouvert (47) et qui débouche dans le premier tuyau (4) en amont du point où ladite liaison (46) est interrompue, ou qui débouche dans l'extrémité supérieure dudit récipient (1) ; on centrifuge de nouveau le récipient (1), à basse vitesse ; on envoie du fluide au corps de déplacement (17) de manière à transférer la couche supérieure obtenue par centrifugation au troisième récipient latéral (43) ; on interrompt l'envoi du fluide et on interrompt la liaison entre le récipient (1) et le troisième récipient latéral (43).

7. Procédé pour diviser le sang en constituants en centrifugeant du sang présent dans un récipient compressible (1) et en transférant dans un mode stérile au moins deux de trois constituants (34, 35, 36) stratifiés par ledit processus de centrifugation dans des récipients latéraux séparés (2, 3) respectivement raccordés à la partie supérieure du récipient de sang (1) au moyen d'un premier tuyau ouvert ou pouvant être ouvert (4) et d'un deuxième tuyau pouvant être ouvert (5), dans lequel le deuxième tuyau (5) comprenant une partie prolongatrice (8) qui se prolonge vers le fond du récipient (1) sur une distance prédéterminée, mesurée à partir de la surface supérieure limite dudit récipient (1), et l'orifice (9) de ladite partie prolongatrice (8) étant placé soit au-dessus, soit au-dessous de la couche centrale (36) des trois couches (34, 35, 36) précitées qui existent après la centrifugation, et en interrompant la communication entre le récipient de sang (1) et lesdits récipients latéraux (2, 3) après le transfert du constituant du sang au récipient latéral respectif (2, 3), caractérisé par les phases consistant à :
avant la centrifugation, placer le récipient de sang (1) et un corps de déplacement (17) pouvant être actionné par fluide dans une cassette (12, 13) qui renferme ledit récipient (1) et ledit corps de déplacement (17) et ayant une ouverture pour établir une liaison fluidique avec le corps de déplacement (17) et les tuyaux (4, 5) reliant le récipient de sang (1) aux récipients latéraux (2, 3), ledit corps de déplacement (17) étant disposé en contact direct avec le récipient (1), le long d'un côté de celui-ci et parallèlement à ce côté ;
après centrifugation, ouvrir la deuxième liaison (6) et, dans le cas d'un premier tuyau pouvant être ouvert (4), également la première liaison (10) et actionner le corps de déplacement (17) avec un fluide pour transférer simultanément le constituant lourd et le constituant léger aux récipients latéraux respectifs (2, 3).

8. Dispositif qui peut être placé dans une cuve de centrifugation, pour la centrifugation et le transport stérile ultérieur d'au moins deux constituants d'un liquide stratifiés obtenus à la suite de la centrifugation d'un liquide (du sang ou une fraction sanguine) présent dans un récipient compressible (1) à des récipients latéraux (2, 3) raccordés à la partie supérieure du récipient compressible (1) au moyen de tuyaux (4, 5), caractérisé en ce que ledit dispositif comprend un cassette de récipient rigide ou semi-rigide (12, 13) qui renferme ledit récipient (1), et un corps de déplacement (17) actionné par fluide étant disposé en appui direct contre un côté du récipient (1) et parallèlement à ce côté, ladite cassette (12, 13) ayant une ouverture pour le passage de raccordements au corps de déplacement (17) et au récipient (1) avec ses récipients latéraux (2, 3), en ce que l'un des raccordements du récipient compressible (1) comprend une partie prolongatrice (8) qui se prolonge vers le fond du récipient (1) sur une distance prédéterminée, mesurée à partir de la surface limite supérieure dudit récipient (1), et en ce que le dispositif peut être séparé de la centrifugeuse avant le transfert des constituants à partir du récipient (1).

9. Dispositif selon la revendication 8, caractérisé en ce que l'aire sur laquelle la surface de déplacement (17) s'appuie contre le récipient de sang est sensiblement égale à la moitié de l'aire dudit récipient.

10. Dispositif selon la revendication 8 ou 9, caractérisé en ce que la résistance de la paroi du corps de déplacement (17) à la dilatation varie de telle manière que le déplacement du contenu du récipient de sang (1) commence à partir du fond dudit récipient.

11. Dispositif selon une ou plusieurs des revendications 8 à 10, caractérisé en ce que le corps de déplacement (17) possède son épaisseur de paroi la plus faible à son extrémité inférieure et en ce que ladite épaisseur de paroi croît avec la distance à cette extrémité.

12. Dispositif selon une ou plusieurs des revendications 8 à 11, caractérisé en ce que seul le côté du corps de déplacement (17) qui regarde vers le récipient de sang a une épaisseur de paroi variable.

13. Dispositif selon une ou plusieurs des revendications 8 à 12, caractérisé en ce que le corps de déplacement (17) est fait d'une matière élastique d'un type qui revient à sa forme d'origine lorsqu'il n'est plus influencé par une pression de fluide.

14. Dispositif selon une ou plusieurs des revendications 8 à 13, caractérisé en ce que le corps de déplacement (17) et le récipient (1) ont une paroi limite commune.
